# EUROPEAN PATENT APPLICATION

(11) **EP 4 621 636 A1**
(43) Date of publication of application: **24.09.2025**
(21) Application number: 24164070.5
(22) Date of filing: 18.03.2024
(51) Int. Cl.: G06K 7/00, A61B 90/98, G06K 7/10

(54) **SYSTEM AND METHOD FOR RFID TAG DATA AGGREGATION OF NESTED RFID-TAGGED MEDICAL DEVICES VIA TWO-STEP READING AND FILTERING**

(71) Applicant: Becton, Dickinson and Company, Franklin Lakes, NJ 07417-1880 (US)
(72) Inventor: LUXEMBOURG, David, 38000 Grenoble (FR); MESSAOUD, Mourad, 38000 Grenoble (FR)
(74) Representative: Germain Maureau

(57) **Abstract**

Provided herein is a system and method for reading a RFID-tagged nest (204) and a plurality of RFID-tagged medical devices (100), for purposes of data aggregation. The method includes conveying a RFID-tagged nest to a reading location inside of an RFID shielding chamber (410) and reading the RFID-tagged nest and RFID-tagged medical devices via a RFID reading system (400) including RFID antenna(s) (405) and a RFID reader (404). In performing the reading, a controller (402) having software stored therein operates the RFID reading system at a low power to perform a first reading and at a high power to perform a second reading. Any EPCs retrieved from the first reading are filtered using a first filter, to remove any EPCs having the second EPC format. Any EPCs retrieved from the second reading are filtered using a second filter, to remove any EPCs having the first EPC format. EPCs of the nest and medical devices are then aggregated.

## Description

### BACKGROUND OF THE INVENTION

### Field of the Invention

The present disclosure relates generally to RFID-tagged medical injection devices retained in packaging including a tub and nest and, more particularly, to a system and method for reading RFID-tagged nests and RFID-tagged medical injection devices within the nest, and for aggregating data from the RFID-tagged nests and RFID-tagged medical injection devices.

### Description of Related Art

Medical injection devices, such as syringes, are used in a variety of environments for administering fluids, such as medications or other drugs, to a patient. Syringes may be provided as prefilled or pre-fillable syringes, which provide the convenience of rapidly delivering the liquid therein to a patient without the need to first aspirate the medication from another container and meter its volume. A syringe will typically include a syringe barrel having a distal end and a proximal end. A needle is connected at the distal end for injecting fluid into the patient and a plunger assembly is inserted through the proximal end that is movable within the barrel to force fluid out from the syringe through the needle.

Components of the syringes as described above often need to be transported from one site to another site during or after manufacturing. For instance, syringe barrels (which, as used herein, may be understood to include the barrel, the needle, and a needle cover) may be transported from a manufacturing site to a site at which the barrels may be filled with a "medicinal fluid," which can refer to a medication or another therapeutic agent used for treatment of chronic or acute conditions, as are known in the art. The filling of the syringe barrels is an automated process that is performed by a fill and finish machine, which dispenses fluid into the syringe barrels in an automated fashion.

During transportation and filling, the syringe barrels may be supported by a holding device, such as a tray or "nest." As known in the art, a nest can be a plate-shaped tray configured to support multiple medical containers in an upright orientation. Nests can comprise multiple "chimneys" aligned according to predetermined rows or a predetermined pattern, with each chimney defining a receptacle or opening configured to receive one medical container. When inserted into and through the receptacle, the medical container is held in the upright orientation in a direction substantially orthogonal to the nest. The nest is usually placed inside a box-shaped tub with an open top, which can be sealed by a sealing cover. The sealing cover can then be removed when the syringes are to be filled with a medicinal fluid by the fill and finish machine.

In an effort to automate the manufacturing of syringes (including filling of the syringes with medicinal fluid) and provide for further traceability of the syringes from the manufacturing process until the final labeling, the final use, or the disposal of the medical devices, it is becoming more common for such syringes to implement RFID tags (including a RFID chip and a RFID antenna) thereon that provide for remote identification and tracing of the syringes. The RFID tag of each syringe includes an electronic product code (EPC) that is specific to that syringe, to enable identification of each syringe.

As relates to the manufacturing process, RFID tags may be integrated into the syringes (e.g., in the needle shield thereof) at the site of initial manufacturing, with the tagged syringes (i.e., RFID-tagged syringe barrels) then loaded into a nest and tub for subsequent shipping. Additionally, the retention of each RFID-tagged syringe within a nest is stored on a nest RFID tag that is secured onto the nest. Thus, the RFID-tagged syringes and the RFID-tagged nest may provide an "aggregation" of data including the unique EPC of each RFID-tagged syringe and a full listing of the syringes included in an RFID-tagged nest, as stored on the nest RFID tag. Knowing an EPC of each syringe (barrel), a fill and finish machine may later be controlled to dispense an appropriate type and quantity of medicinal fluid into each syringe barrel retained within the nest.

As regards the reading of an RFID-tagged nest and the reading of RFID-tagged syringes in a nest (either during manufacturing/packaging or during later identification and tracing of the syringes) for data aggregation, such reading is often performed sequentially for a number of tub-packaged nests/syringes that are in close proximity to one another. The reading of the individual RFID-tagged syringes may require a high power on an RFID reader, while the reading of the RFID-tagged nests may require a lower power on the RFID reader. It is recognized that the need for a high power on the RFID reader for reading of the RFID-tagged syringes can lead to issues of "cross-reading," where the RFID tags on the syringes and/or nest of an adjacent tub may accidentally be read along with the RFID tags on the syringes and/or nest of a target tub, and that this cross-reading can lead to improper/incorrect aggregation data being read.

In an effort to prevent such cross-reading, it is known to perform a reading of the RFID-tagged nest and syringes within a closed environment that provides electromagnetic shielding (e.g., a Faraday Cage), with the RFID-tagged nest and syringes within a tub being read in the closed environment. With proper shielding, it is possible to use RFID reader antennas at high power to read only the RFID tags for the tub within the closed environment, while eliminating the potential for cross-reading of RFID tags in other tubs. However, it is recognized that it may be difficult to guarantee that the shielding provided is sufficient to prevent all RF leaks during reading of the RFID tags within a targeted tub, so as to eliminate the potential for such cross-reading between adjacent tubs.

Accordingly, a need exists in the art for a system and method by which the RFID-tagged nest and syringes within a targeted tub may be read for purposes of data aggregation, while eliminating the potential for the cross-reading of RFID tags in adjacent tubs. The system and method would desirably be capable of aggregating data from the RFID tags of a nest and its associated syringes, without the requirement for a perfectly shielded environment being provided when reading each tub.

### SUMMARY OF THE INVENTION

Provided herein is a method of performing a reading of each of a RFID-tagged nest and a plurality of RFID-tagged medical devices contained in the RFID-tagged nest. The method comprises conveying a RFID-tagged nest retaining a plurality of RFID-tagged medical devices therein, via a conveyor system, to a reading location inside of an RFID shielding chamber, the RFID-tagged nest comprising a RFID nest tag having a nest EPC associated therewith and each of the plurality of RFID-tagged medical devices comprising an RFID device tag integrated therewith having a unique device EPC associated therewith, with the nest EPC having a first EPC format and the device EPCs having a second EPC format. The method also includes providing a RFID reading system comprising a RFID antenna positioned at the reading location, within the RFID shielding chamber, and a RFID reader operably connected with the RFID antenna. The method further includes controlling the RFID reading system, via a controller and software programmed on a processor thereof, to read the RFID-tagged nest and each of the plurality of RFID-tagged medical devices, so as to retrieve the nest EPC and the device EPCs, wherein controlling the RFID reading system comprises setting an output power of the RFID reader to the RFID antenna at a first power, to perform a first reading, and setting an output power of the RFID reader to the RFID antenna at a second power higher than the first power, to perform a second reading. The method still further includes filtering, via the RFID reader or the controller, any EPCs retrieved from the first reading using a first filter, to remove any EPCs having the second EPC format, and filtering, via the RFID reader or the controller, any EPCs retrieved from the second reading using a second filter, to remove any EPCs having the first EPC format.

In accordance with some aspects of the disclosure, the RFID-tagged nest and the RFID-tagged medical devices are packaged in a tub.

In accordance with some aspects of the disclosure, the method includes conveying additional tubs along the conveyor system, with each additional tub including an additional RFID-tagged nest retaining additional RFID-tagged medical devices therein.

In accordance with some aspects of the disclosure, filtering EPCs retrieved from the first reading using the first filter removes device EPCs of the plurality of RFID-tagged medical devices contained in the RFID-tagged nest that were read during the first reading at the first power, to isolate the nest EPC read from the RFID-tagged nest at the reading location.

In accordance with some aspects of the disclosure, filtering EPCs retrieved from the second reading using the second filter removes the nest EPC from the RFID-tagged nest at the reading location and/or nest EPCs from the additional RFID-tagged nests located outside of the RFID shielding chamber that were read during the second reading at the second power, to isolate the device EPCs read from the plurality of RFID-tagged medical devices contained in the RFID-tagged nest at the reading location.

In accordance with some aspects of the disclosure, the method includes aggregating the nest EPC of the RFID-tagged nest at the reading location and the device EPCs of the plurality of RFID-tagged medical devices contained in the RFID-tagged nest.

In accordance with some aspects of the disclosure, aggregation of the nest EPC and the device EPCs associates the RFID-tagged medical devices with the RFID-tagged nest.

In accordance with some aspects of the disclosure, the first power is tuned or set to enable reading of the RFID nest tag from the RFID-tagged nest at the reading location, but not enable the reading of the RFID nest tags from the additional RFID-tagged nests outside of the RFID shielding chamber.

In accordance with some aspects of the disclosure, the second power is tuned or set to enable reading of the RFID device tags from the plurality of RFID-tagged medical devices at the reading location, but not enable the reading of RFID device tags from the additional RFID-tagged medical devices outside of the RFID shielding chamber.

In accordance with some aspects of the disclosure, in performing the reading of the RFID-tagged nest and of each of the plurality of RFID-tagged medical devices, the method includes operating the RFID reader to receive a trigger input signal from a controller or sensor associated with the RFID reader, electromagnetically coupling the RFID antenna to the RFID-tagged nest and each of the plurality of RFID-tagged medical devices responsive to receiving of the trigger signal, and performing the reading of the RFID-tagged nest and the plurality of RFID-tagged medical devices upon the electromagnetic coupling.

Also provided herein is a system for performing a reading of a RFID-tagged nest and a plurality of RFID-tagged medical devices contained within the RFID-tagged nest, the RFID-tagged nest including a RFID nest tag having a nest EPC associated therewith and each of the plurality of RFID-tagged medical devices comprising an RFID device tag integrated therewith having a unique device EPC associated therewith, with the nest EPC having a first EPC format and the device EPCs having a second EPC format. The system includes a conveyor system configured to convey a plurality of tubs along a conveying path, each of the plurality of tubs including a RFID-tagged nest therein that retains a plurality of RFID-tagged medical devices therein. The system also includes a RFID shielding chamber positioned at a reading location along the conveying path that is configured to receive a respective tub therein, and a RFID reading system comprising a RFID antenna positioned at the reading location and within the RFID shielding chamber and a RFID reader operably connected with the RFID antenna and configured to perform a reading of the RFID nest tag and the RFID device tags upon a respective tub passing the RFID antenna at the reading location. The system further includes a controller operably connected with the RFID reading system, the controller including a processor programmed with software to send first control signals to the RFID reading system setting an output power of the RFID reader to the RFID antenna at a first power, to perform a first reading, send second control signals to the RFID reading system setting an output power of the RFID reader to the RFID antenna at a second power higher than the first power, to perform a second reading. The RFID reader or the controller is further configured to filter any EPCs retrieved from the first reading using a first filter, to remove any EPCs having the second EPC format, and filter any EPCs retrieved from the second reading using a second filter, to remove any EPCs having the first EPC format.

In accordance with some aspects of the disclosure, the processor is configured to aggregate the nest EPC of the RFID-tagged nest at the reading location and the device EPCs of the plurality of RFID-tagged medical devices contained in the RFID-tagged nest.

In accordance with some aspects of the disclosure, aggregation of the nest EPC and the device EPCs associates the RFID-tagged medical devices with the RFID-tagged nest.

In accordance with some aspects of the disclosure, in filtering EPCs retrieved from the first reading using the first filter, the processor is configured to remove device EPCs of the plurality of RFID-tagged medical devices contained in the RFID-tagged nest that were read during the first reading at the first power, to isolate the nest EPC read from the RFID-tagged nest at the reading location.

In accordance with some aspects of the disclosure, in filtering EPCs retrieved from the second reading using the second filter, the processor is configured to remove the nest EPC from the RFID-tagged nest at the reading location and/or nest EPCs from the RFID-tagged nests located outside of the RFID shielding chamber that were read during the second reading at the second power, to isolate the device EPCs read from the plurality of RFID-tagged medical devices contained in the RFID-tagged nest at the reading location.

In accordance with some aspects of the disclosure, the RFID reader is configured to tune or set the first power to enable reading of the RFID nest tag from the RFID-tagged nest at the reading location, but not enable the reading of the RFID nest tags from the additional RFID-tagged nests in tubs outside of the RFID shielding chamber.

In accordance with some aspects of the disclosure, the RFID reader is configured to tune or set the second power to enable reading of the RFID device tags from the plurality of RFID-tagged medical devices in the tub at the reading location, but not enable the reading of RFID device tags from the additional RFID-tagged medical devices in tubs outside of the RFID shielding chamber.

In accordance with some aspects of the disclosure, the RFID shielding chamber comprises a faraday cage configured to provide electromagnetic field shielding.

In accordance with some aspects of the disclosure, the system further includes at least one trigger sensor configured to detect when a respective tub is at the reading location, wherein for each tub on the conveyor system, the at least one trigger sensor is configured to transmit a trigger signal to the RFID reader to cause the RFID reader to read the RFID nest tag and the RFID device tags of the RFID-tagged nest and the plurality of RFID-tagged medical devices in the respective tub in response to detecting that the tub is at the reading location.

In accordance with some aspects of the disclosure, the plurality of RFID-tagged medical devices comprise a plurality of syringe barrels.

### BRIEF DESCRIPTION OF THE DRAWINGS

FIG. 1A is a perspective view of a RFID-tagged syringe, with which embodiments of the disclosure may be implemented;
FIG. 1B is an exploded view of the syringe of FIG. 1A;
FIG. 1C is a side view of a needle shield included in the syringe of FIG. 1A;
FIG. 2 is a perspective view of packaging, including a tub and nest, for storing and shipping a plurality of medical components, such as the syringe of FIGS. 1A and 1B, with which embodiments of the disclosure may be implemented;
FIG. 3 is a top view of the nest of FIG. 2;
FIG. 4 illustrates an environment in which a method of reading a plurality of RFID-tagged nests and RFID-tagged syringes may be performed, according to a non-limiting embodiment described herein;
FIG. 5 is a diagram of non-limiting embodiments or aspects of components of one or more devices and/or one or more systems of FIG. 4; and
FIG. 6 is a flowchart of a process for reading a RFID-tagged nest and RFID-tagged medical devices in the nest, for purposes of data aggregation, according to a non-limiting embodiment described herein.

### DESCRIPTION OF THE INVENTION

The following description is provided to enable those skilled in the art to make and use the described embodiments contemplated for carrying out the invention. Various modifications, equivalents, variations, and alternatives, however, will remain readily apparent to those skilled in the art. Any and all such modifications, variations, equivalents, and alternatives are intended to fall within the spirit and scope of the present invention.

For purposes of the description hereinafter, the terms "upper", "lower", "right", "left", "vertical", "horizontal", "top", "bottom", "lateral", "longitudinal", and derivatives thereof shall relate to the invention as it is oriented in the drawing figures. However, it is to be understood that the invention may assume various alternative variations, except where expressly specified to the contrary. It is also to be understood that the specific devices illustrated in the attached drawings, and described in the following specification, are simply exemplary embodiments of the invention. Hence, specific dimensions and other physical characteristics related to the embodiments disclosed herein are not to be considered as limiting.

In the present disclosure, the distal end of a component or of a device means the end furthest away from the hand of the user and the proximal end means the end closest to the hand of the user, when the component or device is in the use position, i.e., when the user is holding a syringe in preparation for or during use. Similarly, in this application, the terms "in the distal direction" and "distally" mean in the direction toward the distal tip of the syringe, and the terms "in the proximal direction" and "proximally" mean in the direction opposite the direction of the distal tip of the syringe.

As used herein, the terms "communication" and "communicate" may refer to the reception, receipt, transmission, transfer, provision, and/or the like of information (e.g., data, signals, messages, instructions, commands, and/or the like). For one unit (e.g., a device, a system, a component of a device or system, combinations thereof, and/or the like) to be in communication with another unit means that the one unit is able to directly or indirectly receive information from and/or transmit information to the other unit. This may refer to a direct or indirect connection that is wired and/or wireless in nature. Additionally, two units may be in communication with each other even though the information transmitted may be modified, processed, relayed, and/or routed between the first and second unit. For example, a first unit may be in communication with a second unit even though the first unit passively receives information and does not actively transmit information to the second unit. As another example, a first unit may be in communication with a second unit if at least one intermediary unit (e.g., a third unit located between the first unit and the second unit) processes information received from the first unit and communicates the processed information to the second unit. In some non-limiting embodiments or aspects, a message may refer to a network packet (e.g., a data packet and/or the like) that includes data. It will be appreciated that numerous other arrangements are possible.

As used herein, the term "computing device" may refer to one or more electronic devices that are configured to directly or indirectly communicate with or over one or more networks. A computing device may be a mobile or portable computing device, a desktop computer, a server, and/or the like that is specifically configured to provide one or more of the features and/or functions described herein. Furthermore, the term "computer" may refer to a specifically configured computing device that includes the necessary components to receive, process, and output data according to aspects described herein, and normally includes a display, a processor, a memory, an input device, and a network interface. A "computing system" may include one or more computing devices or computers. An "application" or "application program interface" (API) refers to computer code or other data sorted on a computer-readable medium that may be executed by a processor to facilitate the interaction between software components, such as a client-side front-end and/or server-side back-end for receiving data from the client. An "interface" refers to a generated display, such as one or more graphical user interfaces (GUIs) with which a user may interact, either directly or indirectly (e.g., through a keyboard, mouse, touchscreen, etc.). Further, multiple computers, e.g., servers, or other computerized devices directly or indirectly communicating in the network environment may constitute a "system" or a "computing system".

As used herein, the term "communication network" may refer to one or more wired and/or wireless networks. For example, a communication network may include a cellular network (e.g., a long-term evolution (LTE) network, a third generation (3G) network, a fourth generation (4G) network, a fifth generation (5G) network, a code division multiple access (CDMA) network, etc.), a public land mobile network (PLMN), a local area network (LAN), a wide area network (WAN), a metropolitan area network (MAN), a telephone network (e.g., the public switched telephone network (PSTN)), a private network, an ad hoc network, an intranet, the Internet, a fiber optic-based network, a cloud computing network, and/or the like, and/or a combination of these or other types of networks.

It will be apparent that systems and/or methods, described herein, can be implemented in different forms of hardware, firmware, or a combination of hardware and software. The actual specialized control hardware or software code used to implement these systems and/or methods is not limiting of the implementations. Thus, the operation and behavior of the systems and/or methods are described herein without reference to specific software code, it being understood that software and hardware can be designed to implement the systems and/or methods based on the description herein.

No aspect, component, element, structure, act, step, function, instruction, and/or the like used herein should be construed as critical or essential unless explicitly described as such. Also, as used herein, the articles "a" and "an" are intended to include one or more items, and may be used interchangeably with "one or more" and "at least one." Furthermore, as used herein, the term "set" is intended to include one or more items (e.g., related items, unrelated items, a combination of related and unrelated items, etc.) and may be used interchangeably with "one or more" or "at least one." Where only one item is intended, the term "one" or similar language is used. Also, as used herein, the terms "has," "have," "having," or the like are intended to be open-ended terms. Further, the phrase "based on" is intended to mean "based at least partially on" unless explicitly stated otherwise.

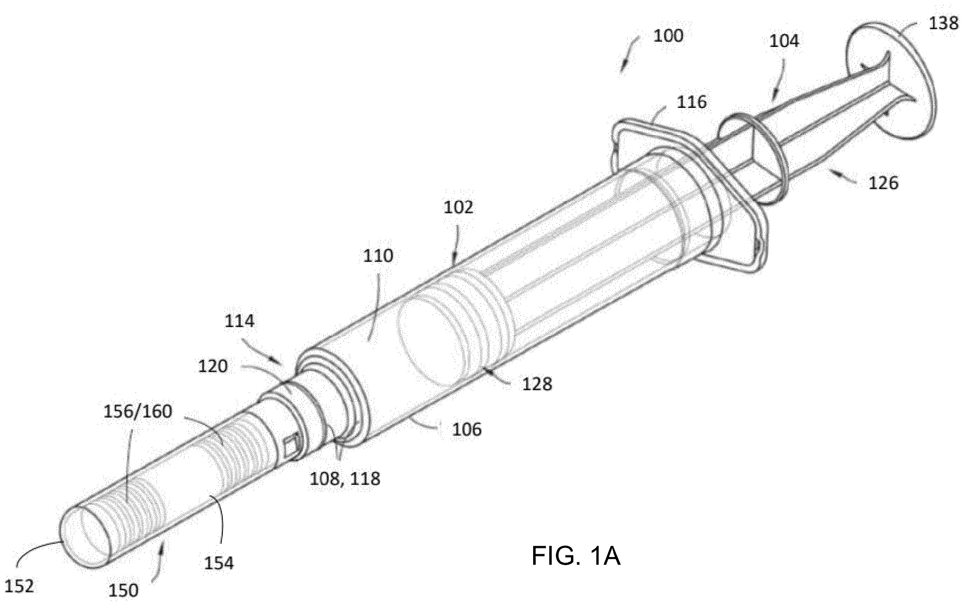

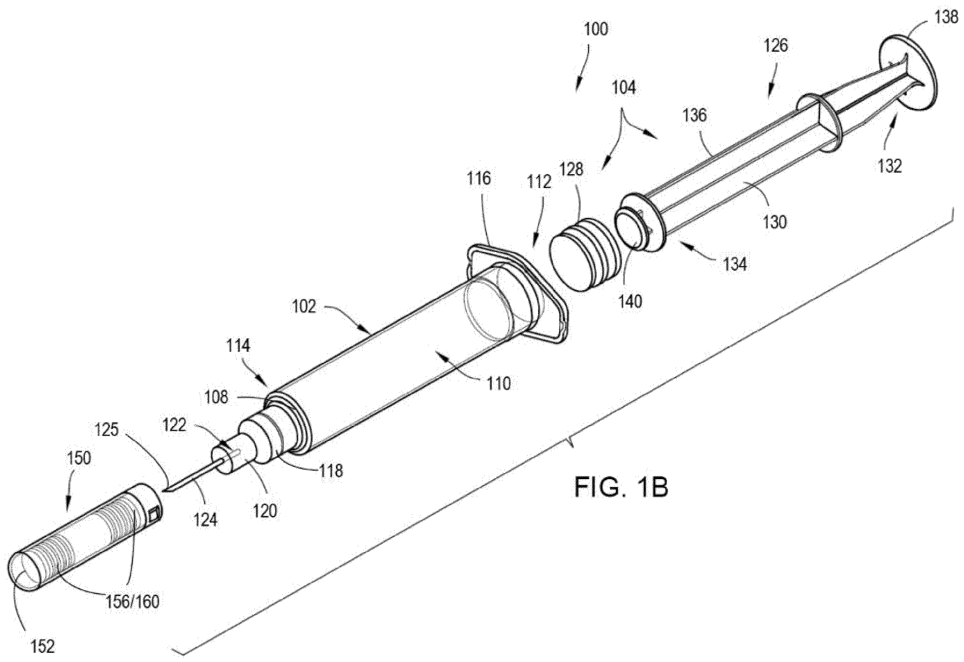

Referring to FIGS. 1A and 1B, shown is a non-limiting embodiment of a RFID-tagged medical device 10 with which aspects or embodiments of the disclosure may be implemented. While the medical device is shown and described hereafter as a syringe ("syringe 100"), it is recognized that other RFID-tagged medical devices, including other medical injection devices (e.g., auto-injectors), may be utilized with the system and method of the disclosure described in detail here below.

As shown in FIGS. 1A and 1B, the syringe 100 generally includes a syringe barrel assembly 102 (hereafter "syringe barrel 102") and a plunger assembly 104. The plunger assembly 104 is movable within the syringe barrel 102 along a longitudinal axis to an advanced position to facilitate administering of an injectable fluid (e.g., medication) to a patient, for example. The syringe barrel 102 is formed of a generally cylindrical outer wall 106 and an end member 108 that collectively define a chamber 110 for retaining fluid therein. The syringe barrel 102 includes an open proximal end 112 configured to receive the plunger assembly 104 therein and a distal end 114 at which end member 108 is positioned. The proximal end 112 of the syringe barrel 102 may include a flange 116 to facilitate handling and positioning of the syringe 100 and to maintain the relative position of the syringe barrel 102 with respect to the plunger assembly 104 during medication administration. At the distal end 114, the end member 108 may include a shoulder 118 which narrows with respect to the cylindrical outer wall 106, as well as a hub portion 120 extending out distally from shoulder 118. The hub portion 120 is formed as a partially hollow member that defines a channel 122 therethrough in fluid communication with the chamber 110. A needle 124 is attached to the hub portion 120 within channel 122, such as by being glued or otherwise secured to the hub portion 120, with the needle 124 extending distally from the hub 120 out to a distal needle tip 125.

The plunger assembly 104 of syringe 100 is formed of an elongate plunger rod 126 (more generally "plunger 126," as used hereafter) and a plunger head or stopper 128. The plunger 126 may include a main body 130 extending between a plunger proximal end 132 and a plunger distal end 134. In some embodiments, the main body 130 may include a plurality of elongate vanes or walls 136 extending axially along a length thereof between the plunger proximal end 132 and the plunger distal end 134. A thumb press 138 is positioned at the plunger proximal end 132 that may be engaged by a thumb (or other finger) of the user to apply a distally directed force to the plunger assembly 104 to move the plunger 126 with respect to the syringe barrel 102. In some embodiments, a flanged extension member 140 (e.g., disc-shaped flange) is positioned at the plunger distal end 134 that is configured to mate with the stopper 128. In other embodiments, the plunger distal end 134 may include a female receptacle formed therein that is configured to receive and connect to a protrusion (e.g., pin) extending out proximally from the stopper, with the protrusion and receptacle engaging via threading, for example.

As shown in FIGS. 1A and 1B, a needle shield 150 of syringe barrel 102 (i.e., of the syringe barrel assembly may be coupled to the hub portion 120 and/or shoulder 118 to protect the needle 124. According to aspects of the disclosure, the needle shield 150 may be composed of a rigid outer casing 152 that provides structure to the needle shield 150 and a compliant inner casing or "jupe" 154 that surrounds the needle 124 (when needle shield 150 is coupled to syringe barrel 102). In various embodiments, the outer casing 152 may be made of a rigid material such as polypropylene (PP), polyethylene (PE), polyethylene terephthalate (PET), polystyrene (PS) or polycarbonate (PC), while the inner casing 154 is made of a deformable material such as rubber (e.g., butyl rubber, isoprene rubber, latex rubber, silicone rubber) or a thermoplastic elastomer (TPE), as non-limiting examples.

According to aspects and embodiments of the disclosure, the needle shield 150 may include an RFID tag 156 integrated therein that allows for identification and/or tracking of the syringe 100. That is, the RFID tag 156 may include or store information thereon regarding the contents of the syringe 100 (i.e., the medication or drug included therein, if a prefilled syringe) and the manufacturing history of the syringe to an associated reader, so as to enable tracking of the syringe 100. Accordingly, the RFID tag 156 is considered to generally include thereon an electronic product code (EPC) that is specific to the syringe 100. According to aspects or embodiments of the disclosure, the RFID tag 156 may be integrated into the needle shield 150, such as by being inlaid or otherwise disposed between the outer casing 152 and the inner casing 154 of the needle shield 150. According to other embodiments, the RFID tag 156 may be applied to an outer surface of the needle shield 150. In each of these embodiments, the RFID tag 156 conforms to the surface(s) or wall(s) to or within which it is disposed, such that when the RFID tag 156 is integrated with/on a cylindrical component such as the needle shield 150, the RFID tag 156 will have a curvature that matches that of the needle shield 150.

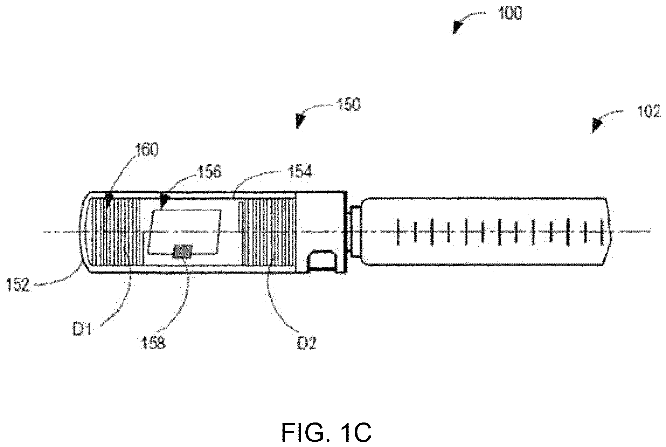

According to embodiments, and as shown in FIG. 1C, the RFID tag 156 includes an RFID chip 158 connected to an RFID dipole coupling element or antenna 160. The antenna 160 of RFID tag 156 may include two legs or dipole elements D1, D2, with the RFID chip 158 disposed between extremities of the poles D1, D2, such as being centered therebetween. In some embodiments, the RFID tag 156 may be positioned and oriented within needle shield 150 such that a dipole axis, A_{D}, of the RFID tag 156 is approximately aligned with a longitudinal axis, Ac, of the needle shield 150 (and a longitudinal axis, As, of the syringe 100), such that more strict quality controls may be implemented during manufacturing/assembly of the syringe 100 and the cap 150 thereof.

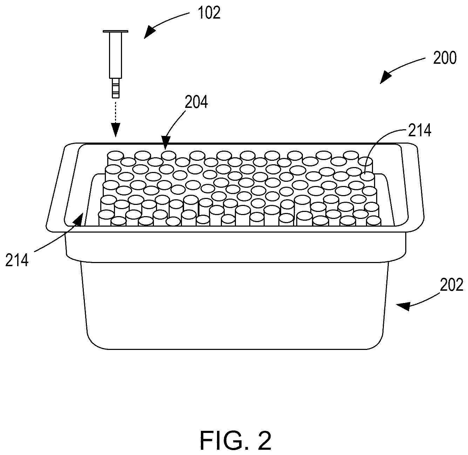

Referring to FIG. 2, shown is a non-limiting embodiment of packaging 200 that may be used for the packaging and transporting of a plurality of medical components therein, such as syringes 100 or syringe barrel assemblies 102 thereof shown in FIGS. 1A-1C. The packaging 200 includes a container or tub 202 in which a nest 204 may be housed, with the nest 204 configured to retain a plurality of syringes 100 or syringe barrels 102 therein. The packaging 200 may also include a sealing cover (not shown) that may be applied over the tub 202 after the nest 204 is packaged therein, to provide a sterile environment for storing and shipping the syringes 100 or syringe barrels 102.

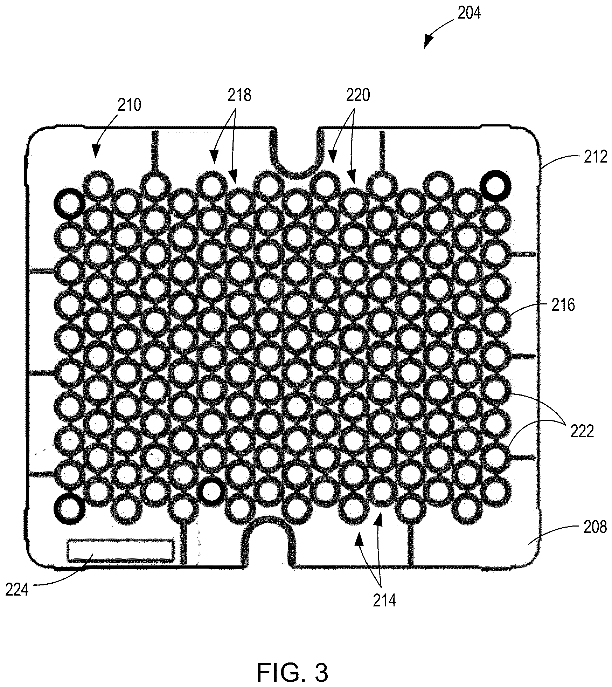

As shown in FIG. 2 and also in FIG. 3, an exemplary nest 204 that may be retained in tub 202 comprises a support plate 208 having a first or upper surface 210, a second or lower surface (now shown), and a peripheral edge 212 extending about the support plate 208. In some examples, the perimeter of support plate 208 is substantially rectangular in shape, while in other embodiments, the support plate 208 may be square. The nest 204 furthers comprise tubular walls, members, or ridges - referred to hereafter as "chimneys 214" - that protrude outward and upward from a plane defined by the upper surface 210 of the support plate 208. Each chimney 214 can include a cylindrical wall 216 that defines a receptacle 218 configured to receive a syringe barrel 102 therein. The receptacle 218 includes a top opening at a top end of the chimney 214 and a bottom opening at a bottom end of the chimney 214, with the receptacle sized to receive the cylindrical outer wall 106 of barrel 102 therein and the flange 116 of barrel 102 resting against an upper edge of the chimney 214. As shown best in FIG. 3, according to some aspects of the disclosure, the nest 204 can be configured to include one-hundred sixty (160) chimneys 214 for receiving syringe barrels 102, although it is recognized that another number of chimneys 214 may be provided on nest 204 that is dependent on the size of the syringe barrels 102 retained therein (e.g., 1-3 mL syringe barrels, 5 mL syringe barrels, 10 mL syringe barrels, etc.). The chimneys 214 may be considered as being arranged in a plurality of columns 220 (e.g., 16 columns, in the embodiment of FIG. 3), with each of the columns 220 including a plurality of "seats" 222 that correspond to the individual chimneys 214 (e.g., 10 seats in each column, with 160 seats total, in the embodiment of FIG. 3).

According to aspects of the disclosure, nest 204 includes an RFID tag 224 secured thereon, such as by being glued on the upper surface 210 of support plate 208, adjacent a corner thereof. The RFID tag 224 allows for identification of the nest 204. That is, the RFID tag 224 may include or store thereon a EPC that is specific to the nest 204, with it recognized that each specific nest 204 will have associated therewith the particular RFID-tagged syringe barrels 102 that are retained in the nest 204. According to a non-limiting embodiment, the EPC of the nest 204 and the EPCs of the RFID-tagged syringe barrels 102 retained thereby may be used for an initial aggregation and/or registration purpose, with the aggregation/registration data them being subsequently used for the filling/dispensing of a medication or drug into the syringe barrels 102 (if a prefilled syringe) during a fill and finish process.

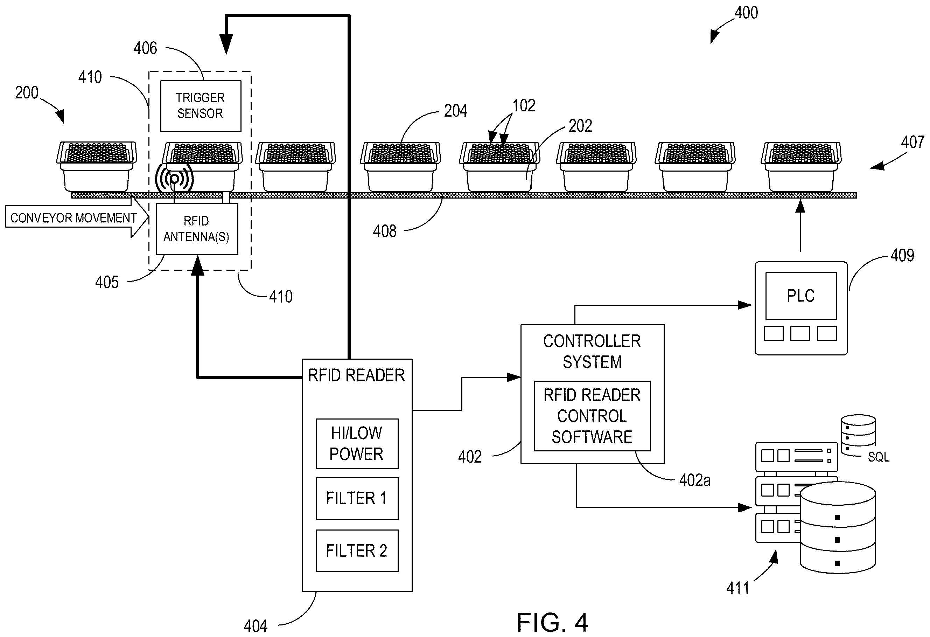

Referring now to FIG. 4, a manufacturing and reading environment or system 400 is illustrated within which or by which the RFID-tagged nest of a packaging (such as the RFID-tagged nest 204 of packaging 200 of FIG. 2 and FIG. 3) and the RFID-tagged syringe barrels contained in the nest may be read, in accordance with an aspect of the disclosure. As shown in FIG. 4, a plurality of packagings 200 (each including a tub 202 and nest 204 with syringe barrels 102 retained therein) may be provided to system 400. According to aspects of the disclosure, reading of the RFID-tagged nests 204 and the RFID-tagged syringe barrels 102 contained in the tubs 202 may be implemented as part of a process for aggregating data on the RFID-tagged nests 204 and their associated RFID-tagged syringe barrels 102 retained therein, which may be used to associate RFID-tagged syringe barrels 102 retained within each nest 204. As indicated above, "syringe barrels 102" may be understood to refer to an assembly of the cylindrical outer wall 106, end member 108, shoulder 118, hub portion 120, needle 124, and needle shield 150 (including RFID tag 156).

As shown in FIG. 4 system 400 may include a controller 402, a RFID reader 404 having one or more RFID antennas 405 (collectively a "RFID reading system"), a trigger sensor 406, a conveyor system 407 (including a conveyor 408 and programmable logic controller (PLC) 409), a RF shielding chamber 410, and a database system 411.

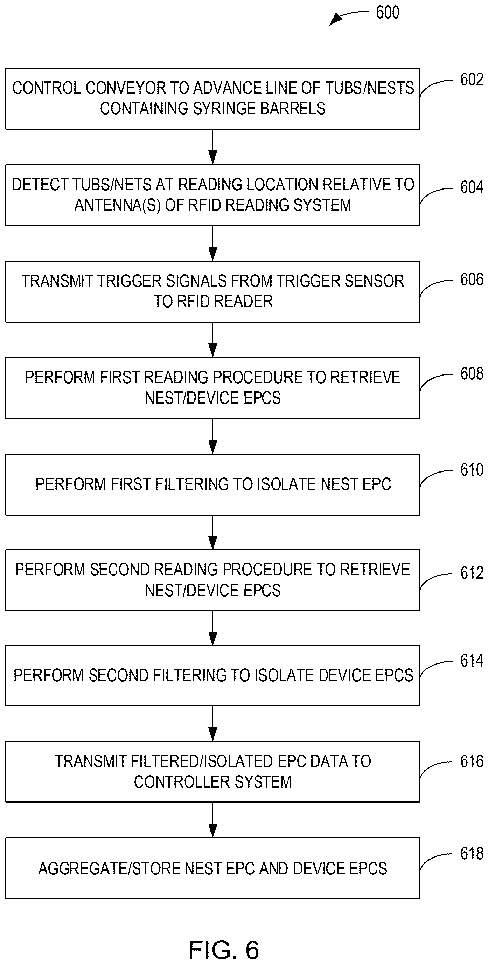

Controller 402 may include one or more devices capable of receiving information and/or data from RFID reader 404, trigger sensor 406, conveyor system 407, and/or database system 411 (e.g., via a communication network, via a direct wired and/or wireless connection, etc.) and/or communicating information and/or data to RFID reader 404, trigger sensor 406, conveyor system 407, and/or database system 411 (e.g., via communication network, via a direct wired and/or wireless connection, etc.). For example, controller 402 may include a computing device, such as a server, a group of servers, and/or other like devices. In some non-limiting embodiments or aspects, controller 402 may include middleware, such as Message Queuing Telemetry Transport (MQTT) protocol-based middleware, and/or the like, configured to manage communication with and/or control of operations of RFID reader 404, trigger sensor 406, conveyor system 407 (i.e., PLC 409 of conveyor system 407, etc.), and/or database system 411. In particular, and as explained in further detail below regarding FIG. 6, software/middleware 402a of controller 402 may send/push control signals ("recipes") to RFID reader 404 for controlling operation thereof.

RFID reader 404 may include one or more devices capable of receiving information and/or data from controller 402, trigger sensor 406, conveyor system 407, and/or database system 411 (e.g., via a communication network, via a direct wired and/or wireless connection, etc.) and/or communicating information and/or data to controller 402, trigger sensor 406, conveyor system 407, and/or database system 411 (e.g., via communication network, via a direct wired and/or wireless connection, etc.).

RFID reader 404 may include a passive RFID reader, an active RFID reader, or any combination thereof. RFID reader 404 may be configured to read - from the RFID tags 224 on the nests 204 and the RFID tags 156 on syringe barrels 102 (within tubs 202) in the production line - EPCs associated with the nests 204 and syringe barrels 102. For example, RFID reader 404 may be configured to attempt to read an RFID tag 224 on a nest 204 and RFID tags 156 on syringe barrels 102 within the nest 204 in response to receiving a trigger signal from trigger sensor 406.

An RFID antenna 405 (which may be provided as a near field antenna or other near field coupling element, but is referred to hereafter generally as "antenna 405") of RFID reader 404 may be located proximate to conveyor 408 such that, as conveyor 408 moves the tubs 202 along the production line, the tubs 202 are moved one-by-one to be adjacent to (and/or to be paused adjacent to for a predetermined period of time) antenna 405 of RFID reader 404 at a "reading location", and then moved past antenna 405 of RFID reader 404. While only a single antenna 405 is illustrated in FIG. 4, it is recognized that a plurality of antennas 405 may be utilized with RFID reader 404, in order enable reading of the RFID tags 156, 224.

Trigger sensor 406 may include one or more devices capable of receiving information and/or data from controller 402, RFID reader 404, conveyor system 407, and/or database system 411 (e.g., via a communication network, via a direct wired and/or wireless connection, etc.) and/or communicating information and/or data to controller 402, RFID reader 404, conveyor system 407, and/or database system 411 (e.g., via communication network, via a direct wired and/or wireless connection, etc.).

Trigger sensor 406 may include at least one sensor (e.g., an optical sensor, a laser sensor, etc.) configured to detect or be trigged when an individual tub 202 is in a predetermined location relative to antenna 405 of the RFID reader 404 (e.g., when a tub 202 is at the reading location, adjacent antenna 405). Trigger sensor 406 may be located proximate or adjacent to antenna 405 of RFID reader 404 and/or conveyor 408 and/or at a position relative to antenna 405 of RFID reader 404 and/or conveyor 408 at which, when a tub 202 is in the predetermined location relative to antenna 405 of the RFID reader 404, the tub 202 is within the field of detection or view of trigger sensor 406 such that trigger sensor 406 is triggered or detects the tub 202. In response to being triggered or detecting a tub 202 in the predetermined location relative to antenna 405 of the RFID reader 404, trigger sensor 406 may be configured to communicate or transmit a trigger signal to RFID reader 404 to trigger or cause RFID reader 404 to attempt to read a RFID tag 224 on the nest 204 and RFID tags 156 on syringe barrels 102 within tub 202.

Conveyor system 407 may include one or more devices capable of receiving information and/or data from controller 402, RFID reader 404, trigger sensor 406, and/or database system 411 (e.g., via a communication network, via a direct wired and/or wireless connection, etc.) and/or communicating information and/or data to controller 402, RFID reader 404, trigger sensor 406, and/or database system 411 (e.g., via communication network, via a direct wired and/or wireless connection, etc.).

Conveyor system 407 may include a conveyor 408 (e.g., a motorized belt conveyor, a motorized roller conveyor, a motorized chain conveyor, a motorized trolley conveyor, etc.) that conveys tubs 202 along a path (e.g., linear path). Each of the tubs 202 may be retained on the conveyor 408, with the tubs 202 held in position by mechanical force or another suitable means. Conveyor 408 may be configured to individually move (e.g., move one-by-one, etc.) tubs 202 in the production line adjacent to and past antenna 405 of RFID reader 404. For example, tubs 202 may be arranged on conveyor 408, and/or conveyor 408 may be configured to move tubs 202 together in a same direction such that tubs 202 are moved one-by-one adjacent to (and/or paused adjacent to for a predetermined period of time), and moved then past, antenna 405 of RFID reader 404.

The PLC 409 of conveyor system 407 is configured to control one or more operations of conveyor system 407, including conveyor 408. In some embodiments, the PLC 409 may be configured to track a sequential order of the tubs 202 in the production line (on conveyor 408), with such tracking performed via one or more shift registers and/or sensors, according to existing manufacturing techniques.

The RF shielding chamber 410 may be positioned adjacent the conveyor 408 at the reading location. The chamber 410 is sized to receive a respective tub 202 therein, as well as the antenna 405 of the RFID reading system. The chamber 410 is configured to provide a closed environment within which the RFID tag 224 on a nest 204 and the RFID tags 156 on syringe barrels 102 within the nest 204 can be read. That is, the chamber 410 may function as a Faraday cage that provides electromagnetic field shielding about the tub 202 and antenna 405, so as to minimize/prevent potential "cross-reading" from occurring in system 400 - i.e., the RFID reader 404 (and antenna 405) reads only the nest RFID tag 224 and device RFID tags 156 on the nest 204 and syringe barrels 102 of a tub 202 that is within chamber 410, and not the RFID tags of nests/syringe barrels within adjacent tubs 202 located outside of the chamber 410.

Database system 411 may include one or more devices capable of receiving information and/or data from controller 402, RFID reader 404, trigger sensor 406, and/or conveyor system 407 (e.g., via a communication network, via a direct wired and/or wireless connection, etc.) and/or communicating information and/or data to controller 402, RFID reader 404, trigger sensor 406, and/or conveyor system 407 (e.g., via communication network, via a direct wired and/or wireless connection, etc.). In some non-limiting embodiments or aspects, database system 411 includes one or more local databases (e.g., local to and/or implemented by controller 402, etc.). In other non-limiting embodiments or aspects, database system 411 includes one or more external databases (e.g., external to and/or not implemented by controller 102, etc.). Database system 411 may be configured to store data on each RFID-tagged nest 204 (i.e., the EPC of each nest RFID tag 224), along with data on the RFID-tagged syringe barrels 102 retained in each nest 204 (i.e., the EPCs of the device RFID tags 156) and the ordering/locations of the RFID-tagged syringe barrels 102 retained in each nest 204, as explained in further detail below.

The number and arrangement of devices shown in FIG. 4 is provided as an example. There can be additional devices, fewer devices, different devices, or differently arranged devices than those shown in FIG. 4. Furthermore, two or more devices shown in FIG. 4 can be implemented within a single device, or a single device shown in FIG. 4 can be implemented as multiple, distributed devices. Additionally, or alternatively, a set of devices (e.g., one or more devices, etc.) of system 400 can perform one or more functions described as being performed by another set of devices of system 400.

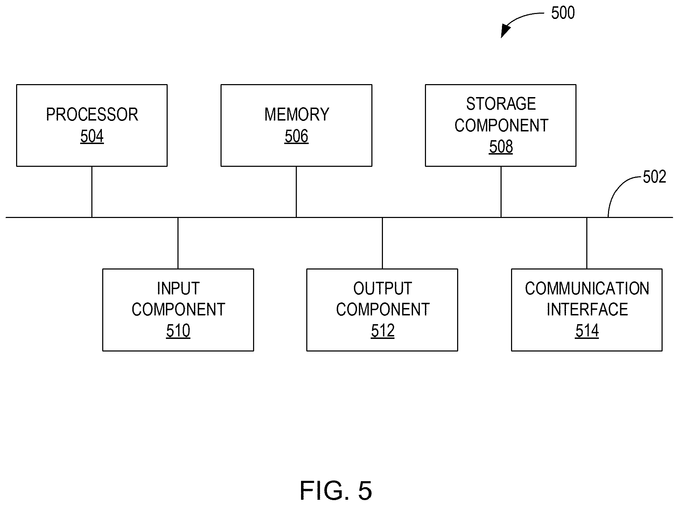

Referring now to FIG. 5, FIG. 5 is a diagram of example components of a device 500. Device 500 may correspond to one or more devices of controller 402, RFID reader 404 (e.g., one or more devices of a system of RFID reader 404, etc.), trigger sensor 406 (e.g., one or more devices of a system of trigger sensor 406, etc.), one or more devices of conveyor system 407, an/or one or more devices of database system 411. In some non-limiting embodiments or aspects, one or more devices of controller 402, RFID reader 404 (e.g., one or more devices of a system of RFID reader 404, etc.), trigger sensor 406 (e.g., one or more devices of a system of trigger sensor 406, etc.), and/or one or more devices of conveyor system 407 one or more devices of database system 411, may include at least one device 500 and/or at least one component of device 500. As shown in FIG. 5, device 500 may include bus 502, processor 504, memory 506, storage component 508, input component 510, output component 512, and communication interface 514.

Bus 502 may include a component that permits communication among the components of device 500. In some non-limiting embodiments or aspects, processor 504 may be specifically configured to perform one or more of the mixing aspects described in hardware, software, or a combination of hardware and software. For example, processor 504 may include a processor (e.g., a central processing unit (CPU), a graphics processing unit (GPU), an accelerated processing unit (APU), etc.), a microprocessor, a digital signal processor (DSP), and/or similar processing component (e.g., a field-programmable gate array (FPGA), an application-specific integrated circuit (ASIC), etc.) that can be programmed to perform a function. Memory 506 may include random access memory (RAM), read-only memory (ROM), and/or another type of dynamic or static storage device (e.g., flash memory, magnetic memory, optical memory, etc.) that stores information and/or instructions for use by processor 504.

Storage component 508 may store information and/or software related to the operation and use of device 500. For example, storage component 508 may include a hard disk (e.g., a magnetic disk, an optical disk, a magneto-optic disk, a solid state disk, etc.), a compact disc (CD), a digital versatile disc (DVD), a floppy disk, a cartridge, a magnetic tape, and/or another type of computer-readable medium, along with a corresponding drive.

Input component 510 may include a component that permits device 500 to receive information, such as via user input (e.g., a touch screen display, a keyboard, a keypad, a mouse, a button, a switch, a microphone, etc.). Additionally or alternatively, input component 510 may include a sensor for sensing information (e.g., a global positioning system (GPS) component, an accelerometer, a gyroscope, an actuator, etc.). Output component 512 may include a component that provides output information from device 500 (e.g., a display, a speaker, one or more light-emitting diodes (LEDs), etc.).

Communication interface 514 may include a transceiver-like component (e.g., a transceiver, a separate receiver and transmitter, etc.) that enables device 500 to communicate with other devices, such as via a wired connection, a wireless connection, or a combination of wired and wireless connections. Communication interface 514 may permit device 500 to receive information from another device and/or provide information to another device. For example, communication interface 514 may include an Ethernet interface, an optical interface, a coaxial interface, an infrared interface, a radio frequency (RF) interface, a universal serial bus (USB) interface, a Wi-Fi^{®} interface, a cellular network interface, and/or the like.

Device 500 may perform one or more processes described herein. Device 500 may perform these processes based on processor 504 (e.g., a central processing unit (CPU), a graphics processing unit (GPU), etc.) executing software instructions stored by a computer-readable medium, such as memory 506 and/or storage component 508. A computer-readable medium (e.g., a non-transitory computer-readable medium) is defined herein as a non-transitory memory device. A non-transitory memory device includes memory space located inside of a single physical storage device or memory space spread across multiple physical storage devices.

Software instructions may be read into memory 506 and/or storage component 508 from another computer-readable medium or from another device via communication interface 514. When executed, software instructions stored in memory 506 and/or storage component 508 may cause processor 504 to perform one or more processes described herein. Additionally or alternatively, hardwired circuitry may be used in place of or in combination with software instructions to perform one or more processes described herein.

Memory 506 and/or storage component 508 may include data storage or one or more data structures (e.g., a database, etc.). Device 500 may be capable of receiving information from, storing information in, communicating information to, or searching information stored in the data storage or one or more data structures in memory 506 and/or storage component 508.

The number and arrangement of components shown in FIG. 5 are provided as an example. In some non-limiting embodiments or aspects, device 500 may include additional components, fewer components, different components, or differently arranged components than those shown in FIG. 5. Additionally or alternatively, a set of components (e.g., one or more components) of device 500 may perform one or more functions described as being performed by another set of components of device 500.

According to aspects of the disclosure, it is recognized that reading of the RFID tags 224 of the nests 204 and the RFID tags 156 of the syringe barrels 102 by the RFID reader 404 can be used to aggregate nest and "device" (i.e., syringe/syringe barrel) data, so as to associate particular syringe barrels 102 with a particular nest 204 (and tub 202),. The aggregated data acquired on a RFID-tagged nest 204 and the RFID-tagged syringe barrels 102 retained therein may be stored, and in some examples, may be subsequently used in order to properly register the RFID-tagged syringe barrels 102 and facilitate filling and finishing thereof via a fill and finish machine that dispenses a controlled amount and type of medicament into the syringe barrels 102 (for providing pre-filled syringes).

Referring now to FIG. 6, a flowchart is provided for a non-limiting embodiment or aspect of a method 600 for reading RFID-tagged nests and RFID-tagged medical devices contained in the RFID-tagged nests, for purposes of implementing an aggregation strategy for associating RFID-tagged nests and RFID-tagged medical devices retained therein. The method is performed in a manner that reduces or eliminates the possibility of the cross-reading of RFID tags of nests/syringe barrels in adjacent tubs other than a target tub, thereby ensuring accurate data aggregation.

In some non-limiting embodiments or aspects, one or more of the steps of process 600 may be performed (e.g., completely, partially, etc.) by controller 402 (e.g., one or more devices of controller 402, etc.). In some non-limiting embodiments or aspects, one or more of the steps of process 600 may be performed (e.g., completely, partially, etc.) by another device or a group of devices separate from or including controller 402, such as RFID reader 404 (e.g., one or more devices of a system of RFID reader 404, etc.), trigger sensor 406 (e.g., one or more devices of a system of trigger sensor 406, etc.), conveyor system 407 (e.g., PLC 409), and/or database system 411.

According to aspects of the disclosure, and as explained in further detail below, controller 402 includes software 402a thereon (e.g., that is stored in memory 506 and/or run by processor 504 of controller 402) via which control signals or "recipes" are sent/pushed to RFID reader 404 to implement/perform a two-step reading process by which RFID-tagged nests and RFID-tagged medical devices contained in the RFID-tagged nests are read (and filtered), for purposes of aggregating EPC data from an RFID-tagged nest and RFID-tagged medical devices retained therein, for associating the medical devices with the nest..

As shown in FIG. 6, at step 602, process 600 includes controlling a conveyor to start a production line of packages - i.e., of tubs/nests and syringe barrels retained therein. For example, controller 402 may control conveyor system 407 to cause conveyor 408 to start conveyor movement to move the tubs 202/nests 204 in the production line adjacent to and past antenna 405 of RFID reader 404, i.e., to the reading location. As an example, controller 402 (e.g., middleware of controller 402, etc.) may send a signal to conveyor system 407 (e.g., the PLC 409 of conveyor system 407, etc.) to cause conveyor system 407 to control conveyor 408 to start conveyor movement. In such an example, controller 402 (e.g., middleware of controller 402, etc.) may send a signal to arm the RFID reader 404.

As shown in FIG. 6, at step 604, process 600 includes detecting a tub in a predetermined location relative to an antenna of a RFID reader. For example, for each tub/nest of the plurality of tubs 202/nests 204 in the production line, trigger sensor 406 may detect when that tub 202/nest 204 is in a predetermined location relative to antenna 405 of RFID reader 404 (i.e., at the reading location). As previously described, when a tub 202 is at the reading location, the tub 202 may be positioned within RF shielding chamber 410, to provide a shielded environment for reading of the RFID-tagged nest 204 and RFID-tagged syringe barrels 102 contained within the tub 202.

As shown in FIG. 6, at step 606, process 600 includes transmitting a trigger signal to a RFID reader. For example, for each tub/nest of the plurality of tubs 202/nests 204 in the production line, trigger sensor 406 may, in response to detecting that the tub 202/nest 204 is at a predetermined location relative to antenna 405 of RFID reader 404 in the production line (i.e., at the reading location), transmit a trigger signal to RFID reader 404 to trigger or cause RFID reader 404 to read the nest RFID tag 224 of the nest 204.

As shown in FIG. 6, at steps 608-614, process 600 performs a multi-step reading and filtering process that, along with the presence of RF shielding chamber 410, functions to eliminate the potential for cross-reading to occur, where the nest RFID tag 224 and/or device RFID tags 156 in (adjacent) tubs other than the target tub 202 may inadvertently be read, which can lead to an inaccurate data aggregation. As explained in further detail below, the multi-step reading and filtering process performs a pair of readings at different powers and performs a pair of data filtering processes, in order to isolate a nest EPC from the nest RFID tag 224 and device EPCs from the device RFID tags 156 and provide for accurate data aggregation.

At step 608, process 600 performs a first reading. The first reading may be initiated in response to receiving the trigger signal from trigger sensor 406, indicating that a target tub 202 is at the reading location. The controller 402 (i.e., software 402a thereof) initiates the first reading procedure by transmitting a command to RFID reader 404 that sets a (low) output power of the RFID reader 404, with the RFID reader 404 outputting the set power to the antenna 405 to enable reading of the nest RFID tag 224 of the nest 204 within tub 202 currently at the reading location 108. The power output to the antenna 405 by the RFID reader 404 as part of the first reading procedure may be characterized as a "low power" that is tuned/set to enable reading of the nest RFID tag 224 of the nest 204 within the chamber 410, but not strong enough to read the nest RFID tag 224 of any nests 204 outside of the chamber 410 (i.e., of nests 204 in tubs 202 outside the chamber 410). The low power output provided to the antenna 405 by the RFID reader 404 is designed to electromagnetically couple the antenna 405 to the nest RFID tag 224 of the nest 204 in the target tub 202 at the reading location, with radio waves from the antenna 405 activating the nest RFID tag 224. Responsive to receiving the radio waves, the nest RFID tag 224 may transmit a (backscattered) signal that includes an EPC associated with that nest 204, back to the antenna 405, to be read by RFID reader 404. In addition to reading the nest RFID tag 224, it is recognized that the low power output provided to the antenna 405 by the RFID reader 404 may also electromagnetically couple the antenna 405 to at least some of the device RFID tags 156 retained in the nest 204, such that at least some of the device RFID tags 156 retained in the nest 204 are read by RFID reader 404, with the device RFID tags 156 transmitting (backscattered) signals that include EPCs associated with the syringe barrels 102.

The process 600 continues at step 610 with the performing of a first filtering designed to isolate the reading of the nest RFID tag 224 from the readings of any device RFID tags 156, as performed in the first reading procedure of step 608. The filtering may be performed by a processor (e.g., processor 504) that, as shown in FIG. 4 (see "Filter 1"), may be included in the RFID reader 404, but it is recognized that the filtering could instead be performed by controller 402 or by another processing device. According to aspects of the disclosure, the filtering operates to filter out or otherwise remove any readings (i.e., EPCs) of the device RFID tags 156 from the reading (i.e., EPC) of the nest RFID tag 224 (within the target tub 202). This filtering of the device EPCs from the nest EPC is enabled based on the format of the device EPCs being distinct/different from the nest EPC. For example, the device EPCs and the nest EPC may each include a header value (e.g., the first eight (8) bits of the EPC) and a filter value (e.g., the next three (3) bits of the EPC), but the filter value of the device EPCs differs from the filter value of the nest EPC, such that the device EPCs may be distinguished from the nest EPC. With the filter configured to filter out or otherwise remove EPCs having a filter value matching the filter value of the device EPCs, the nest EPC that is read from nest RFID tag 224 may be isolated.

As shown in FIG. 6, process 600 performs a second reading at step 612. The second reading procedure may be performed after completion of the first reading procedure of step 608 (and before, during, or after the first filtering of step 610). The controller 402 (i.e., software 402a thereof) initiates the second reading procedure by transmitting a command to RFID reader 404 that sets a (high) output power of the RFID reader 404, with the RFID reader 404 outputting the set power to the antenna 405 to enable reading of the device RFID tags 156 of syringe barrels 102 retained in nest 204 and syringe barrels 102 currently at the reading location 108. The power output to the antenna 405 by the RFID reader 404 as part of the second reading procedure may be characterized as a "high power" (i.e., higher than the low power) that is tuned/set to enable reading of the device RFID tags 156 of the syringe barrels 102 within the chamber 410, but not strong enough to read the device RFID tags 156 of any syringe barrels 102 outside of the chamber 410 (i.e., of syringe barrels 102 in nests 202 outside the chamber 410). The high power output provided to the antenna 405 by the RFID reader 404 is designed to electromagnetically couple the antenna 405 to the device RFID tags 156 of the syringe barrels 102 in the target tub 202 at the reading location, with radio waves from the antenna 405 activating the device RFID tags 156. Responsive to receiving the radio waves, the device RFID tags 156 may transmit a (backscattered) signal that includes EPCs associated with each of the syringe barrels 102, back to the antenna 405, to be read by RFID reader 404. In addition to reading the device RFID tags 156, it is recognized that the high power output provided to the antenna 405 by the RFID reader 404 may also electromagnetically couple the antenna 405 to nest RFID tags 224 of nests 204 in adjacent tubs 202 outside of the chamber 410, such that one or more nest RFID tags 224 of adjacent nests 204 are read by RFID reader 404, with the nest RFID tags 224 transmitting (backscattered) signals that include EPCs associated with the nest 204.

The process 600 continues at step 614 with the performing of a second filtering designed to isolate the reading of the device RFID tags 156 from the readings of any nest RFID tags 224, as retrieved in the second reading procedure of step 612. The filtering may be performed by a processor (e.g., processor 504) that, as shown in FIG. 4 (see "Filter 2"), may be included in the RFID reader 404, but it is recognized that the filtering could instead be performed by controller 402 or by another processing device. According to aspects of the disclosure, the filtering operates to filter out or otherwise remove any readings (i.e., EPCs) of nest RFID tags 224 from the readings (i.e., EPC) of the device RFID tags 156 (within the target tub 202). This filtering of nest EPCs from the device EPCs is enabled based on the format of the device EPCs being distinct/different from the nest EPC. As described above, the device EPCs may include a first filter value therein, while the nest EPC includes a different second filter value. With the filter configured to filter out or otherwise remove EPCs having a filter value matching the filter value of the nest EPCs, the device EPCs that are read from device RFID tags 156 may be isolated.

As shown in FIG. 6, process 600 continues at step 616 with filtered information or data that has been read from the nest RFID tag 224 and device RFID tags 156 being transmitted from the RFID reader 404 to controller 402 (e.g., to middleware of controller 402, etc.). That is, the nest EPC and device EPCs from the RFID-tagged nest 204 and RFID-tagged syringe barrels 102 within the target tub 202 that were isolated in the first and second filtering procedures of steps 610 and 614 are provided to controller 402.

As shown in FIG. 6, process 600 continues at step 618, with the controller 402 aggregating the nest EPC and device EPCs received at step 616, and with the aggregated nest EPC and device EPCs being stored. The aggregating of the nest EPC and device EPCs enables associating (for each target tub 202) the device EPCs from each of the RFID-tagged syringe barrels 102 with the nest EPC of the RFID-tagged nest 204.

In some embodiments, the aggregated nest EPC and device EPCs may be provided from the controller 402 to the database system 411, for storage of the aggregated data. The database system 411 may thus store the EPCs of the RFID-tagged syringe barrels 102 retained within an associated nest 204. As previously described, the aggregated data for the RFID-tagged nest 204 and RFID-tagged syringe barrels 102 within a tub 202 may, as a non-limiting example, subsequently be used to facilitate filling and finishing of the syringe barrels 102 by a fill and finish machine that dispenses a controlled amount and type of medicament into the syringe barrels 102 (for providing pre-filled syringes).

It is recognized that the process 600 described above may be performed for each of the plurality of tubs 202, as the tubs 202 are moved along conveyor 608 - with the multi-step reading and filtering process of steps 608-614 being performed upon each tub 202 reaching the reading location along conveyor 608. Thus, aggregated nest EPC and device EPCs may be acquired and stored for the RFID-tagged nest 204 and RFID-tagged syringe barrels 102 within each tub 202.

Beneficially, embodiments of the invention thus provide a system and method by which the RFID-tagged nest and RFID-tagged medical devices within a target container/tub may be read, for purposes of implementing an aggregation strategy for associating the RFID-tagged nest and the RFID-tagged medical devices retained therein. The system and method is capable of aggregating data from the RFID tags of the nest and its associated medical devices, without the requirement for a perfectly shielded environment being provided when reading a tub. The method is performed in a manner that reduces or eliminates the possibility of the cross-reading of RFID tags of nests/syringe barrels in other tubs adjacent the target tub, thereby ensuring accurate data aggregation.

Although the present disclosure has been described in detail for the purpose of illustration based on what is currently considered to be the most practical and preferred embodiments or aspects, it is to be understood that such detail is solely for that purpose and that the present disclosure is not limited to the disclosed embodiments or aspects, but, on the contrary, is intended to cover modifications and equivalent arrangements that are within the spirit and scope of the appended claims. For example, it is to be understood that the present disclosure contemplates that, to the extent possible, one or more features of any embodiment may be combined with one or more features of any other embodiment.

## Claims

1. A method of performing a reading of each of a radio frequency identification (RFID)-tagged nest (204) and a plurality of RFID-tagged medical devices (100) contained in the RFID-tagged nest, the method comprising:
conveying a RFID-tagged nest retaining a plurality of RFID-tagged medical devices therein, via a conveyor system (407), to a reading location inside of an RFID shielding chamber (410), the RFID-tagged nest comprising a RFID nest tag (224) having a nest electronic product code (EPC) associated therewith and each of the plurality of RFID-tagged medical devices comprising an RFID device tag (156) integrated therewith having a unique device EPC associated therewith, with the nest EPC having a first EPC format and the device EPCs having a second EPC format;
providing a RFID reading system (400) comprising:
a RFID antenna (405) positioned at the reading location, within the RFID shielding chamber; and
a RFID reader (404) operably connected with the RFID antenna;
controlling the RFID reading system, via a controller (402) having software programmed on a processor thereof, to read the RFID-tagged nest and of each of the plurality of RFID-tagged medical devices, so as to retrieve the nest EPC and the device EPCs, wherein controlling the RFID reading system comprises:
setting an output power of the RFID reader to the RFID antenna at a first power, to perform a first reading; and
setting an output power of the RFID reader to the RFID antenna at a second power higher than the first power, to perform a second reading;
filtering, via the RFID reader or the controller, any EPCs retrieved from the first reading using a first filter, to remove any EPCs having the second EPC format; and
filtering via the RFID reader or the controller any EPCs retrieved from the second reading using a second filter, to remove any EPCs having the first EPC format.

2. The method of claim 1, wherein the RFID-tagged nest (204) and the RFID-tagged medical devices (100) are packaged in a tub (202).

3. The method of claim 2, further comprising conveying additional tubs (202) along the conveyor system (407), with each additional tub including an additional RFID-tagged nest (204) retaining additional RFID-tagged medical devices (100) therein.

4. The method of claim 3, wherein filtering EPCs retrieved from the first reading using the first filter removes device EPCs of the plurality of RFID-tagged medical devices (100) contained in the RFID-tagged nest (204) that were read during the first reading at the first power, to isolate the nest EPC read from the RFID-tagged nest at the reading location.

5. The method of claim 3, wherein filtering EPCs retrieved from the second reading using the second filter removes the nest EPC from the RFID-tagged nest (204) at the reading location and/or nest EPCs from the additional RFID-tagged nests located outside of the RFID shielding chamber (410) that were read during the second reading at the second power, to isolate the device EPCs read from the plurality of RFID-tagged medical devices (100) contained in the RFID-tagged nest at the reading location.

6. The method of any of claims 1-5, further comprising aggregating the nest EPC of the RFID-tagged nest (204) at the reading location and the device EPCs of the plurality of RFID-tagged medical devices (100) contained in the RFID-tagged nest, and
wherein, optionally, aggregation of the nest EPC and the device EPCs associates the RFID-tagged medical devices (10) with the RFID-tagged nest (204).

7. The method of any of claims 1-6, wherein the first power is tuned or set to enable reading of the RFID nest tag (224) from the RFID-tagged nest (204) at the reading location, but not enable the reading of the RFID nest tags from the additional RFID-tagged nests outside of the RFID shielding chamber (410), and
wherein, optionally, the second power is tuned or set to enable reading of the RFID device tags (156) from the plurality of RFID-tagged medical devices (10) at the reading location, but not enable the reading of RFID device tags from the additional RFID-tagged medical devices outside of the RFID shielding chamber.

8. The method of any of claims 1-7, wherein in performing the reading of the RFID-tagged nest (204) and of each of the plurality of RFID-tagged medical devices (100), the method comprises operating the RFID reader (404) to:
receive a trigger input signal from a controller or sensor (406) associated with the RFID reader;
electromagnetically couple the RFID antenna (405) to the RFID-tagged nest and each of the plurality of RFID-tagged medical devices responsive to receiving of the trigger signal; and
perform the reading of the RFID-tagged nest and the plurality of RFID-tagged medical devices upon the electromagnetic coupling.

9. A system for performing a reading of a RFID-tagged nest (204) and a plurality of RFID-tagged medical devices (100) contained within the RFID-tagged nest, the RFID-tagged nest including a RFID nest tag (224) having a nest electronic product code (EPC) associated therewith and each of the plurality of RFID-tagged medical devices comprising an RFID device tag (156) integrated therewith having a unique device EPC associated therewith, with the nest EPC having a first EPC format and the device EPCs having a second EPC format, the system comprising:
a conveyor system (407) configured to convey a plurality of tubs (202) along a conveying path, each of the plurality of tubs including a RFID-tagged nest therein that retains a plurality of RFID-tagged medical devices therein;
a RFID shielding chamber (410) positioned at a reading location along the conveying path, the RFID shielding chamber configured to receive a respective tub therein;
a RFID reading system (400) comprising:
a RFID antenna (405) positioned at the reading location and within the RFID shielding chamber; and
a RFID reader (404) operably connected with the RFID antenna and configured to perform a reading of the RFID nest tag and the RFID device tags upon a respective tub passing the RFID antenna at the reading location; and
a controller (402) operably connected with the RFID reading system, the controller including a processor (504) programmed with software to:
send first control signals to the RFID reading system setting an output power of the RFID reader to the RFID antenna at a first power, to perform a first reading; and
send second control signals to the RFID reading system setting an output power of the RFID reader to the RFID antenna at a second power higher than the first power, to perform a second reading;
wherein the RFID reader or the processor is further configured to:
filter any EPCs retrieved from the first reading using a first filter, to remove any EPCs having the second EPC format; and
filter any EPCs retrieved from the second reading using a second filter, to remove any EPCs having the first EPC format.

10. The system of claim 9, wherein the processor (504) is programmed to aggregate the nest EPC of the RFID-tagged nest (204) at the reading location and the device EPCs of the plurality of RFID-tagged medical devices (100) contained in the RFID-tagged nest, and
wherein, optionally, aggregation of the nest EPC and the device EPCs associates the RFID-tagged medical devices with the RFID-tagged nest (224).

11. The system of any of claims 9-10, wherein in filtering EPCs retrieved from the first reading using the first filter, the processor (504) is programmed to remove device EPCs of the plurality of RFID-tagged medical devices (100) contained in the RFID-tagged nest (204) that were read during the first reading at the first power, to isolate the nest EPC read from the RFID-tagged nest at the reading location, and
wherein, optionally, in filtering EPCs retrieved from the second reading using the second filter, the processor is programmed to remove the nest EPC from the RFID-tagged nest at the reading location and/or nest EPCs from the RFID-tagged nests located outside of the RFID shielding chamber (410) that were read during the second reading at the second power, to isolate the device EPCs read from the plurality of RFID-tagged medical devices contained in the RFID-tagged nest at the reading location.

12. The system of any of claims 9-11, wherein the RFID reader (404) is configured to tune or set the first power to enable reading of the RFID nest tag (224) from the RFID-tagged nest (204) at the reading location, but not enable the reading of the RFID nest tags from the additional RFID-tagged nests in tubs (202) outside of the RFID shielding chamber (410), and
wherein, optionally, the RFID reader is configured to tune or set the second power to enable reading of the RFID device tags (156) from the plurality of RFID-tagged medical devices (10) in the tub at the reading location, but not enable the reading of RFID device tags from the additional RFID-tagged medical devices in tubs outside of the RFID shielding chamber.

13. The system of any of claims 9-12, wherein the RFID shielding chamber (410) comprises a faraday cage configured to provide electromagnetic field shielding.

14. The system of any of claims 9-13, further comprising at least one trigger sensor (406) configured to detect when a respective tub (202) is at the reading location;
wherein, for each tub on the conveyor system (407), the at least one trigger sensor is configured to transmit a trigger signal to the RFID reader (404) to cause the RFID reader to read the RFID nest tag (224) and the RFID device tags (156) of the RFID-tagged nest (204) and the plurality of RFID-tagged medical devices (100) in the respective tub in response to detecting that the tub is at the reading location.

15. The system of any of claims 9-14, wherein the plurality of RFID-tagged medical devices (100) comprise a plurality of syringe barrels (102).
